# EUROPEAN PATENT APPLICATION

(11) **EP 2 949 222 A1**
(43) Date of publication of application: **02.12.2015**
(21) Application number: 14743342.9
(22) Date of filing: 21.01.2014
(51) Int. Cl.: A23L 1/10, A23L 1/105, C12N 9/26

(54) **METHOD FOR MANUFACTURING STARCH-CONTAINING FOOD PRODUCT, AND ENZYME PREPARATION FOR MODIFYING STARCH-CONTAINING FOOD PRODUCT**

(30) Priority: 24.01.2013 JP 2013010789
(71) Applicant: Ajinomoto Co., Inc., Tokyo 104-8315 (JP)
(72) Inventor: SUZUKI, Yuki, Kawasaki-shi Kanagawa 210-8681 (JP); USUGI, Koudai, Kawasaki-shi Kanagawa 210-8681 (JP)
(74) Representative: Strehl Schübel-Hopf & Partner
(86) International application number: PCT/JP2014/051870
(87) International publication number: WO 2014/115894

(57) **Abstract**

A method of producing a starch-containing food having improved physical properties and taste by using a branching enzyme and an α-glucosidase, and an enzyme preparation for modifying a starch-containing food are provided.

## Description

### Technical Field

The present invention relates to a method of producing a starch-containing food using a branching enzyme and an α-glucosidase and an enzyme preparation for modifying a starch-containing food.

### Background Art

Many foods are composed of various components such as starch, proteins, saccharides, and lipids, and these components constitute the textures of foods in a complex manner. Above all, starch and proteins largely contribute to the textures of foods and a change in starch over time is particularly regarded as important.

When gelatinized starch is left at room temperature or low temperature, water is liberated and the gelatinized starch is hardened. This phenomenon is called retrogradation, and a number of studies have been made with respect to the retrogradation phenomenon of starch. In general, in order to prevent retrogradation, it is necessary to maintain starch at a temperature of 80°C or higher, to quickly dry starch to a water content of 15% or less, or to maintain starch in an alkaline condition of pH 13 or higher. Further, as a method of preventing retrogradation, a method of adding a saccharide (such as glucose, fructose, or liquid sugar), a soy protein, wheat gluten, a fatty acid ester, or a polysaccharide (such as yam or konj ac) to a starch-containing food is generally known, and JP-A-59-2664 describes a method of adding a thickener, a surfactant, or the like. However, according to such a method, the taste is largely changed, and also the effect is unstable, and therefore, it cannot be a sufficient solution.

Further, a method of adding an enzyme is also known as a means for preventing retrogradation. For example, JP-A-58-86050 describes a method of improving cooked rice by cooking rice after mixing polished rice with enzymes such as amylase, protease, and lipase, common salt, and a cyclodextrin. JP-A-60-199355 describes a method of preventing retrogradation of cooked rice by adding an aqueous solution of a saccharifying amylase (β-amylase or glucoamylase) to cooked rice after cooking by spraying. Although attempts to improve the quality of cooked rice have been made by adding various enzyme preparations to rice in this manner, the current situation is that no remarkable effect has been obtained in any of these attempts.

Further, JP-A-2000-236825 describes a method of improving cooked rice by cooking rice mixed with a cluster dextrin, which is a cyclic dextrin produced by a branching enzyme. It is known that an anti-adhesion property and a suppression of retrogradation are imparted to cooked rice by a cluster dextrin.

JP-A-57-132850 reports that by adding a branching enzyme to various foods supplemented with a high starch content, retrogradation is prevented, and also the thickening property or the like is enhanced, and describes Examples in which a branching enzyme was added to bread and Uiro (a sweetened steamed cake made of rice flour), but does not describe that by the addition of a branching enzyme, hardness or elasticity is imparted.

WO 2005/096839 reports that by adding to rice a transglucosidase which is an α-glucosidase as an agent for improving the physical properties of a starch-containing food when rice is cooked, cooked rice which is soft, sticky, and hardly retrograded over time can be obtained, but does not describe that by the addition of an α-glucosidase, hardness or elasticity is imparted.

As described above, techniques for preventing retrogradation or controlling stickiness in a starch-containing food have been disclosed, but there is no case in which a branching enzyme and an α-glucosidase are used in combination as active ingredients for a starch-containing food, and there has been no report that by using these enzymes in combination, "hardness" or "elasticity" can be imparted.

### Disclosure of the Invention

An object of the present invention is to provide a method of producing a starch-containing food having improved physical properties and an enzyme preparation for modifying a starch-containing food. More specifically, an object of the present invention is to provide a method of producing a starch-containing food having a texture which could not be obtained by the single addition of a branching enzyme or an α-glucosidase, for example, "hardness" or "elasticity", by using a branching enzyme and an α-glucosidase in combination, and also to provide an enzyme preparation for modifying a starch-containing food.

The present inventors made intensive studies and as a result, they found that the above object can be achieved by using a branching enzyme and an α-glucosidase in combination, and thus completed the present invention. That is, the present invention is as follows.
(1) A method of producing a starch-containing food, characterized by adding a branching enzyme and an α-glucosidase to a starting material.
(2) The method according to (1), wherein the addition amount of the branching enzyme is from 2.0×10⁻¹⁶ to 4.0×10⁵ U per g of the starting material, and the addition amount of the α-glucosidase is from 1.0×10⁻⁴ to 5.0×10⁷ U per g of the starting material.
(3) The method according to (1) or (2), wherein the addition amount of the branching enzyme is from 4.0×10⁻²⁴ to 40 U per U of the α-glucosidase.
(4) The method according to any one of (1) to (3), wherein the starch-containing food is a cooked rice food or a processed rice product, and the starting material is uncooked rice (non-glutinous rice or glutinous rice).
(5) The method according to any one of (1) to (3), wherein the starch-containing food is a bread or a noodle.
(6) An enzyme preparation for modifying a starch-containing food containing a branching enzyme and an α-glucosidase as active ingredients.
(7) The enzyme preparation according to (6), wherein the content of the branching enzyme is from 4.0×10⁻²⁴ to 40 U per U of the α-glucosidase.

According to the present invention, "hardness" or "elasticity" can be imparted to a starch-containing food, and thus, the quality of the starch-containing food can be improved.

The branching enzyme (EC 2.4.1.18) to be used in the present invention is an enzyme which produces a branching structure of an α-1,6 bond such as amylopectin or glycogen by transferring a part of a 1, 4-α-D-glucan chain to the 6-OH group of a 1,4-α-D-glucan as a receptor. An enzyme for foods which is manufactured by Nagase & Co., Ltd. and named "branching enzyme" is one example.

The α-glucosidase (EC 3.2.1.20) to be used in the present invention is an enzyme which produces α-glucose by hydrolyzing a nonreducing terminal α-1,4-glucosidic bond. Among such α-glucosidases, transglucosidase is preferred. Incidentally, an enzyme commercially available from Amano Enzyme, Inc. under a trade name of "transglucosidase L "Amano"" is one example of the α-glucosidase.

The starting material to be used in the present invention is a food starting material containing starch such as rice, wheat flour, a root tuber such as potato or sweet potato, corn, or the like. The starch-containing food of the present invention is not particularly limited as long as it is a food containing starch, and examples thereof include foods in which starch contributes to the texture and physical properties of the foods. Specifically, representative examples thereof include cooked rice foods (cooked rice (cooked white rice), vinegared rice (rice prepared for sushi), sekihan (glutinous rice steamed with red beans), pilaf, fried rice, rice seasoned and cooked with various ingredients, steamed glutinous rice, rice porridge, risotto, rice balls, sushi, bento (a packed lunch), etc.), processed rice products (senbei crackers (thin rice crackers), okaki crackers (cubic rice crackers), Japanese style confectioneries, rice cakes, etc.), breads and bakeries (plain breads, French breads, etc.), noodles (wheat noodles (udon noodles, Chinese noodles, pasta, etc.), soba (buckwheat) noodles, rice flour noodles, etc.), processed wheat foods (dumpling wrappers, tempura batters, crackers, biscuits, cereals, donuts, etc.), processed corn products (cereals, etc.), and processed foods obtained by using a root tuber such as potato or sweet potato or another vegetable such as corn as a starting material. Among these, cooked rice foods, breads and bakeries, and noodles are particularly preferred. Further, frozen products, aseptically packaged products, retort products, dried products, and canned products thereof are also included.

In the method of producing a starch-containing food of the present invention, as a method of allowing the branching enzyme and the α-glucosidase to act on the starting material, these enzymes may be added and allowed to act on the starting material at any stage until completion of cooking. By taking the case of cooking rice as an example, these enzymes may be added to a soaking liquid in which uncooked rice as a starting material is soaked for the water absorption, or these enzymes may be added after the soaking before cooking the rice. Further, the order of allowing the branching enzyme and the α-glucosidase to act on rice is not particularly limited, and either one of the enzymes may be allowed to act on rice first, and thereafter, the other enzyme may be allowed to act on the rice. However, it is preferred that the two enzymes are allowed to simultaneously act on rice. Further, it does not matter if a starting material usually used for a food is used in combination.

In the present invention, the addition amount of the α-glucosidase may be any as long as the enzyme activity per g of the starting material (in the case of a cooked rice food, per g of uncooked rice as the starting material) is 1.0×10⁻⁴ U or more. However, appropriate is preferably 1.0×10⁻⁴ to 5.0×10⁷ U, more preferably 5.0×10⁻² to 5.0×10⁵ U, further more preferably 1.0×10⁻¹ to 1.0×10⁴ U, particularly preferably 1.0×10 to 1.0×10³ U. Incidentally, in the case where the addition amount of the enzyme is very small, a solution with a measurable concentration of the enzyme may be prepared, and thereafter, the solution may be diluted to be added. For example, when a 1 U/mL solution is prepared and 1 µL thereof is added, the amount of the enzyme is 1×10⁻³ U. Incidentally, with respect to the enzyme activity of the α-glucosidase, the amount of the enzyme which produces 1 µg of glucose in 2.5 mL of a reaction mixture was defined as 1 U (unit) when 1 mL of 0.02 M acetate buffer (pH 5.0) was added to 1 mL of 1 mM α-methyl-D-glucoside, and 0.5 mL of an enzyme solution was added thereto to act at 40°C for 60 minutes.

In the present invention, the addition amount of the branching enzyme may be any as long as the enzyme activity per g of the starting material (in the case of a cooked rice food, per g of uncooked rice as the starting material) is 2.0×10⁻¹⁶ U or more. However, it is preferably from 2.0×10⁻¹⁶ to 4.0×10⁵ U, more preferably from 2.0×10⁻¹⁰ to 4.0×10³ U, further more preferably from 2.0×10⁻⁶ to 4.0×10² U, and particularly preferably from 2.0×10⁻³ to 2.0×10 U. Incidentally, in the case where the addition amount of the enzyme is very small, a solution with a measurable concentration of the enzyme may be prepared, and thereafter, the solution may be diluted and added. For example, when a 1 U/mL enzyme solution is prepared, the solution is diluted 10,000 times to make a 0.0001 U/mL solution, and 1 µL thereof is added so that the amount of the enzyme is 1.0×10⁻⁷ U. Further, the addition amount of the branching enzyme may be any as long it is 4.0×10⁻²⁴ U or more per U of the α-glucosidase, but it is preferably from 4.0×10⁻²⁴ to 40 U, more preferably from 1.0×10⁻¹² to 20 U, further more preferably from 2.0×10⁻⁸ to 2 U, and particularly preferably from 1.0×10⁻⁵ to 2.0×10⁻¹ U. The enzyme activity of the branching enzyme was defined as follows. To 50 µL of 0.1% amylose B (Nacalai Tesque) dissolved in 0.08 M phosphate buffer (pH 7.0), 50 µL of an enzyme solution dissolved in 0.1 M phosphate buffer (pH 7.0) was added, and a reaction was allowed to proceed at 50°C for 30 minutes. Thereafter, 2 mL of an iodine reagent (a liquid obtained by mixing 0.5 mL of 1 N HCl with 0.5 mL of a liquid obtained by dissolving 0.26 g of I₂ and 0.26 g of KI in 10 mL of Milli-Q water, and diluting it to 130 mL) was added thereto, and an absorbance at 660 nm was measured. The amount of the enzyme with which the absorbance at 660 nm was decreased by 1% per minute reaction in the reaction system was defined as 1 U (unit).

The reaction time of each enzyme is not particularly limited as long as it is a time which allows the enzyme to act on starch which is a substrate substance, but is preferably from 5 minutes to 24 hours as a practical time of action. Further, the reaction temperature is also not particularly limited as long as it is within a range capable of maintaining the activity of the enzyme, but it is preferred that the enzyme is allowed to act at 0 to 80°C as a practical temperature. That is, by using these enzymes in a usual cooking and processing process, a sufficient reaction time can be obtained, and "hardness" or "elasticity" can be imparted to the starch-containing food.

The enzyme preparation for modifying a starch-containing food of the present invention contains a branching enzyme and an α-glucosidase. The content of the branching enzyme may be any as long as the content thereof per U of the α-glucosidase is 4.0×10⁻²⁴ U or more, but is preferably from 4.0×10⁻²⁴ to 40 U, more preferably from 1.0×10⁻¹² to 20 U, further more preferably from 2.0×10⁻⁸ to 2 U, and particularly preferably from 1.0×10⁻⁵ to 2.0×10⁻¹ U. This enzyme preparation may further contain another food additive, for example, an excipient such as a dextrin, starch, processed starch, or reducing maltose syrup, a seasoning such as a meat extract, a protein such as a plant protein, gluten, egg white, gelatin, or casein, a protein hydrolysate, a partially degraded protein, an emulsifier, a chelating agent such as a citrate or a polyphosphate, a reducing agent such as glutathione or cysteine, alginic acid, salt water, a fat or oil, a dye, an acidifier, a flavor, or the like in addition to the branching enzyme and the α-glucosidase. The enzyme preparation of the present invention may be in any form selected from a liquid, a paste, a granule, and a powder.

### Brief Description of the Drawings

FIG. 1 shows the results of measurement of the physical properties with respect to hardness and stickiness of cooked rice according to Example 1 of the present invention.
FIG. 2 shows the results of measurement of the physical property with respect to elasticity of cooked rice according to Example 1 of the present invention.
FIG. 3 shows the results of measurement of the physical properties with respect to hardness and stickiness of cooked rice according to Example 1 of the present invention.
FIG. 4 shows the results of measurement of the physical property with respect to elasticity of cooked rice according to Example 1 of the present invention.

### Best Mode for Carrying Out the Invention

Hereinafter, the present invention will be described in more detail with reference to Examples, but the present invention is by no means limited to these Examples.

### Example 1

384 g of commercially available uncooked rice "Koshihikari grown in Niigata Prefecture" (Kitoku Shinryo Co., Ltd.) was rinsed with tap water and soaked in tap water for 1 hour. After water was drained off, the soaked rice was put into a rice cooker (Mitsubishi Electric Corporation, NJ-HS06), and tap water was added thereto in an amount 1.52 times the weight of the uncooked rice. A branching enzyme (3,650 U/g, manufactured by Nagase & Co., Ltd.) (hereinafter referred to as "BE") and an α-glucosidase (transglucosidase L "Amano", 608, 000 U/g, manufactured by Amano Enzyme, Inc.) (hereinafter referred to as "AG") were added thereto and dissolved, and the rice was cooked in the rice cooker. After completion of the cooking, the cooked rice was transferred to a vat, covered with plastic wrap, and cooled in a quick cooler for foods (Miura Co., Ltd., CMJ-40) until the temperature of the cooked rice was decreased to 20°C. Then, the cooked rice was placed in a plastic container with a lid, and stored at room temperature until evaluation was carried out. The amount of each enzyme was as shown in Table 1.

**Table 1**

| Test group | Control group | Test group 1 | Test group 2 | Test group 3 | Test group 4 | Test group 5 | Test group 6 | Test group 7 |
|---|---|---|---|---|---|---|---|---|
| Addition amount of AG (U/g per g of uncooked rice) | - | 190 | - | - | - | - | - | - |
| Addition amount of BE (U/g per g of uncooked rice) | - | - | 2.0E-10 | 2.0E-09 | 2.0E-08 | 2.0E-07 | 2.0E-06 | 2.0E-05 |
| Test group | Test group 8 | Test group 9 | Test group 10 | Test group 11 | Test group 12 | Test group 13 | Test group 14 | Test group 15 |
| Addition amount of AG (U/g per g of uncooked rice) | - | - | - | - | - | 190 | 190 | 190 |
| Addition amount of BE (U/g per g of uncooked rice) | 2.0E-04 | 2.0E-03 | 2.0E-02 | 2.0E-01 | 2.0E+01 | 2.0E-10 | 2.0E-09 | 2.0E-08 |
| Test group | Test group 16 | Test group 17 | Test group 18 | Test group 19 | Test group 20 | Test group 21 | Test group 22 | Test group 23 |
| Addition amount of AG (U/g per g of uncooked rice) | 190 | 190 | 190 | 190 | 190 | 190 | 190 | 190 |
| Addition amount of BE (U/g per g of uncooked rice) | 2.0E-07 | 2.0E-06 | 2.0E-05 | 2.0E-04 | 2.0E-03 | 2.0E-02 | 2.0E-01 | 2.0E+01 |
| Test group | Test group 24 | Test group 25 | Test group 26 | Test group 27 | Test group 28 | Test group 29 | Test group 30 | Test group 31 |
| Addition amount of AG (U/g per g of uncooked rice) | 0.38 | 0.76 | 1.9 | 3.8 | 7.6 | 19 | 38 | 76 |
| Addition amount of BE (U/g per g of uncooked rice) | 4.0E-04 | 8.0E-04 | 2.0E-03 | 4.0E-03 | 8.0E-03 | 2.0E-02 | 4.0E-02 | 8.0E-02 |

The sensory evaluation and physical property evaluation of the cooked rice was carried out after the cooked rice was cooled to room temperature by vacuum cooling immediately after cooking, and thereafter the cooled cooked rice was microwaved. The sensory evaluation was carried out with respect to the following three items: "hardness", "stickiness", and "elasticity". In the sensory evaluation items, the "hardness" represents the strength of stress felt when the cooked rice is chewed and ground, the "stickiness" represents the adhesiveness of the surface of a rice grain, and the "elasticity" represents the strength of repulsive stress, that is, resilience when the cooked rice is chewed. The evaluation was carried out by 5 persons according to a scoring system with scores ranging from -2 to 2 in which the enzyme non-added group (control group) was given a score of 0. The detailed evaluation criteria are shown in Table 2. The results are shown in Table 3.

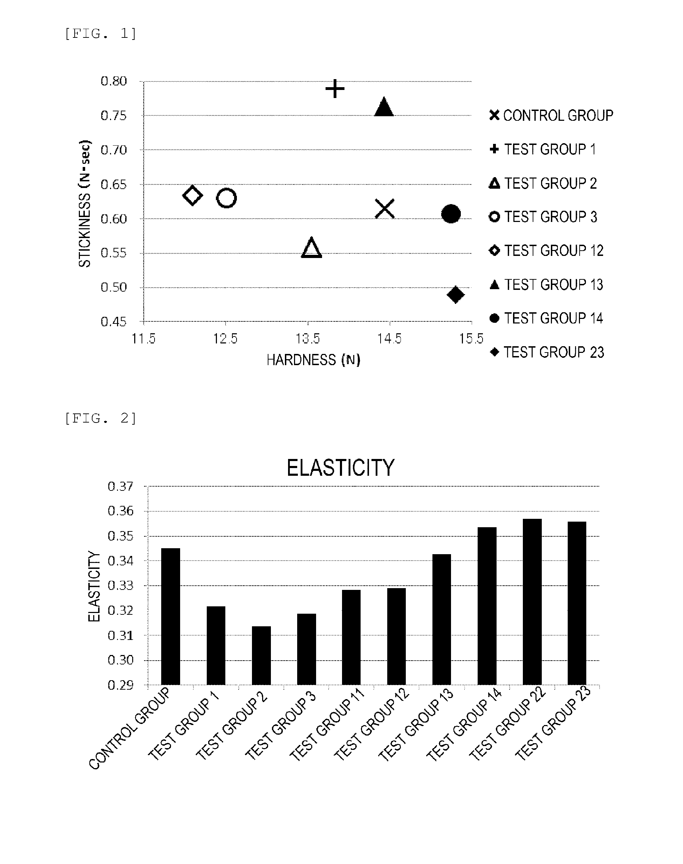

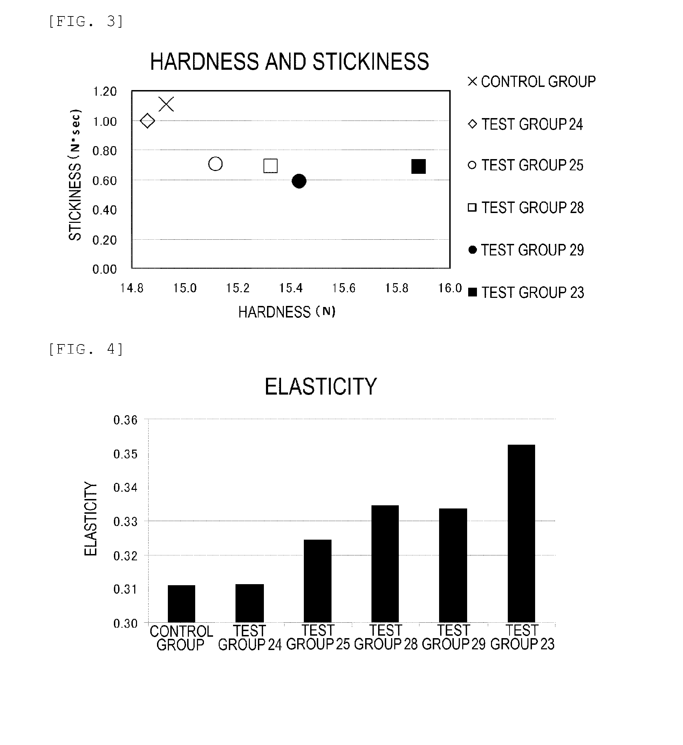

Further, the physical property evaluation was carried out using a texture analyzer of Stable Micro Systems, Ltd. as follows. One grain of cooked rice after cooking was compressed by 90% at 1 mm/s twice using an acrylic cylindrical plunger with a diameter of 3 cm, and the breaking stress at the time of compression by 90% was defined as the hardness, the negative peak area at the time of the first compression by 90% was defined as the stickiness, and the ratio between the breaking distance at the time of the first compression and the breaking distance at the time of the second compression (second time/first time) was defined as the elasticity. The results are shown in FIGS. 1, 2, 3, and 4.

As shown in Table 3 and FIGS. 1, 2, 3, and 4, in the case where only BE (test groups 2 to 12) or only AG (test group 1) was added, the "hardness" of the cooked rice was decreased as compared with that of the control group, and "softness" was imparted. On the other hand, in the case where 2.0×10⁻⁹ U of BE per g of the uncooked rice and 190 U of AG per g of the uncooked rice were used in combination (test groups 14 to 23), although "softness" was imparted by the single addition of each enzyme, surprisingly, "hardness" which is contrary to "softness" was imparted, and moreover, "elasticity" was also imparted. Incidentally, the "hardness" imparted by using BE and AG in combination was different from the hardness due to retrogradation of starch, and was preferred hardness. Further, also in the case where BE and 0.76 U or more of AG per g of the uncooked rice were used in combination, "hardness" different from the hardness due to retrogradation and "elasticity" were imparted in the same manner.

**Table 2**

| | -2 | -1 | 0 | 1 | 2 |
|---|---|---|---|---|---|
| Hardness | Very soft | Soft | There is no difference from control group | Hard | Very hard |
| Stickiness | Stickiness is very low | Stickiness is low | There is no difference from control group | Stickiness is high | Stickiness is very high |
| Elasticity | Elasticity is very low | Elasticity is low | There is no difference from control group | Elasticity is high | Elasticity is very high |

**Table 3**

| | Control group | Test group 1 | Test group 2 | Test group 3 | Test group 4 | Test group 5 | Test group 6 | Test group 7 |
|---|---|---|---|---|---|---|---|---|
| Hardness | 0 | -0.5 | -0.5 | -0.5 | -0.5 | -0.6 | -0.75 | -1 |
| Stickiness | 0 | 0.5 | 0.5 | 0.6 | 0.6 | 0.6 | 1 | 1.25 |
| Elasticity | 0 | 0 | 0 | 0 | 0 | 0 | 0 | 0 |

| | Test group 8 | Test group 9 | Test group 10 | Test group 11 | Test group 12 | Test group 13 | Test group 14 | Test group 15 |
|---|---|---|---|---|---|---|---|---|
| Hardness | -1.2 | -1.5 | -1.75 | -2 | -2 | 0.2 | 0.3 | 0.4 |
| Stickiness | 1.2 | 1.5 | 1.75 | 2 | 2 | 0 | 0 | 0 |
| Elasticity | 0 | 0 | 0 | 0 | 0 | 0 | 0.25 | 0.4 |

| | Test group 16 | Test group 17 | Test group 18 | Test group 19 | Test group 20 | Test group 21 | Test group 22 | Test group 23 |
|---|---|---|---|---|---|---|---|---|
| Hardness | 0.5 | 1 | 1 | 1.2 | 1.5 | 1.75 | 2 | 2 |
| Stickiness | 0 | 0 | 0 | 0 | 0 | 0 | 0 | 0 |
| Elasticity | 0.5 | 0.75 | 1 | 1.25 | 1.5 | 1.75 | 2 | 2 |

| | Test group 24 | Test group 25 | Test group 26 | Test group 27 | Test group 28 | Test group 29 | Test group 30 | Test group 31 |
|---|---|---|---|---|---|---|---|---|
| Hardness | 0.1 | 0.3 | 0.4 | 0.5 | 0.75 | 1 | 1.5 | 1.75 |
| Stickiness | 0 | 0 | 0 | 0 | 0 | 0 | 0 | 0 |
| Elasticity | 0.1 | 0.25 | 0.3 | 0.5 | 0.75 | 1 | 1.5 | 1.75 |

### Example 2

100 g of strong wheat flour (manufactured by Nisshin Flour Milling, Inc.) and 100 g of weak wheat flour (manufactured by Nisshin Flour Milling, Inc.) were mixed with each other, and 86 g of an aqueous solution of 7.5% common salt in which BE (3,650 U/g, manufactured by Nagase & Co., Ltd.) and/or AG (608,000 U/g, manufactured by Amano Enzyme, Inc.) were/was dissolved was added thereto and mixed for 10 minutes using a kneader (manufactured by Kitchen Aid, Inc.). The dough obtained by mixing was gathered up, put into a sealed bag, and left to stand at room temperature for 1 hour (a resting step). The dough was rolled to a thickness of 6 mm using a pasta machine ("R.M.", manufactured by Imperia, Inc.), and then, cut to a width of 6.5 mm ("R-220", manufactured by Imperia, Inc.), whereby an udon noodle was prepared. The thus prepared udon noodle was frozen and stored. The frozen udon noodle was boiled in boiling water for 18 minutes, and then cooled in ice water for 1.5 minutes. Immediately thereafter, the sensory evaluation was carried out. The amount of each enzyme was as shown in Table 4.

**Table 4**

| Test group | Control group | Test group 32 | Test group 33 | Test group 34 |
|---|---|---|---|---|
| Addition amount of AG (U/g per g of flour) | - | - | 125 | 125 |
| Addition amount of BE (U/g per g of flour) | - | 1.0 | - | 1.0 |

The sensory evaluation of the udon noodle was carried out immediately after boiling the noodle. The sensory evaluation was carried out with respect to the following two items: "hardness" and "stickiness". In the sensory evaluation items, the "hardness" represents the strength of stress felt at the start of chewing the noodle, and the "stickiness" represents a force felt as if the teeth were pulled by the noodle. In the same manner as in Example 1, the evaluation was carried out by 5 persons according to a scoring system with scores ranging from -2 to 2 in which the enzyme non-added group (control group) was given a score of 0. The detailed evaluation criteria are shown in Table 5. The results are shown in Table 6.

**Table 5**

| | -2 | -1 | 0 | 1 | 2 |
|---|---|---|---|---|---|
| Hardness | Very soft | Soft | There is no difference from control group | Hard | Very hard |
| Stickiness | Stickiness is very low | Stickiness is low | There is no difference from control group | Stickiness is high | Stickiness is very high |

**Table 6**

| Test group | Control group | Test group 32 | Test group 33 | Test group 34 |
|---|---|---|---|---|
| Hardness | 0 | -0.5 | -0.1 | 2 |
| Stickiness | 0 | 1 | 0 | 1 |

As shown in Table 6, in the case where only BE or AG was added, the "hardness" of the udon noodle was decreased as compared with that of the control group, and "softness" was imparted. On the other hand, in the case where BE and AG were used in combination, not only "stickiness" comparable to the effect imparted by the single addition of BE was imparted, but also "hardness" which is contrary to "softness" was imparted although "softness" was imparted by the single addition of each enzyme in the same manner as in the cooked rice.

### Example 3

To 2 kg of durum wheat flour "DF" (manufactured by Nisshin Flour Milling, Inc.), BE (3,650 U/g, manufactured by Nagase & Co., Ltd.) and/or AG (transglucosidase L "Amano", 608,000 U/g, manufactured by Amano Enzyme, Inc.) were/was added, followed by sufficient mixing. The test groups were the following four test groups: an enzyme non-added group; a BE added group; an AG added group; and a BE and AG added group. To the above mixed starting material, 540 g of tap water was added, and kneading was carried out using a kneader "vacuum mixer VU-2" (manufactured by Okuba Iron Works Co. Ltd.) for 15 minutes (the kneader speedwas set to 100). After completion of the kneading, extrusion noodle making was carried out by a pasta machine "vacuum extruder FPV-2" (manufactured by Nippn Engineering Co., Ltd.) using a 1.8 mm die for long pasta. The extruded noodle strings were dried by a dryer "constant temperature and humidity chamber LH21-13P" (manufactured by Nagano Science Co. , Ltd.), whereby dry pasta was obtained. The dry pasta was boiled in boiling water for 9 minutes, and immediately thereafter, the sensory evaluation was carried out. The amount of each enzyme was as shown in Table 7.

**Table 7**

| Test group | Control group | Test group 35 | Test group 36 | Test group 37 |
|---|---|---|---|---|
| Addition amount of AG (U/g per g of flour) | - | - | 225 | 225 |
| Addition amount of BE (U/g per g of flour) | - | 4.4 | - | 4.4 |

The sensory evaluation of the pasta was carried out immediately after boiling the pasta. The sensory evaluation was carried out with respect to the following two items: "hardness" and "glutinousness". In the sensory evaluation items, the "hardness" represents the strength of stress felt at the start of chewing the noodle, and the "glutinousness" represents a sense of the noodle sticking to the teeth when the pasta is chewed and ground, similarly to the "stickiness". In the same manner as in Example 1, the evaluation was carried out by 5 persons according to a scoring system with scores ranging from -2 to 2 in which the enzyme non-added group (control group) was given a score of 0. The detailed evaluation criteria are shown in Table 8. The results are shown in Table 9.

**Table 8**

| | -2 | -1 | 0 | 1 | 2 |
|---|---|---|---|---|---|
| Hardness | Very soft | Soft | There is no difference from control group | Hard | Very hard |
| Glutinousness | Glutinousness is very low | Glutinousness is low | There is no difference from control group | Glutinousness is high | Glutinousness is very high |
| Stickiness | Stickiness is very low | Stickiness is low | There is no difference from control group | Stickiness is high | Stickiness is very high |

**Table 9**

| Test group | Control group | Test group 35 | Test group 36 | Test group 37 |
|---|---|---|---|---|
| Hardness | 0 | 0 | 0 | 2 |
| Glutinousness | 0 | 1.5 | 2 | 1.5 |

As shown in Table 9, in the case where only BE or AG was added, the "hardness" was comparable to that of the control group, and only "glutinousness" was imparted. On the other hand, in the case where BE and AG were used in combination, "hardness", which was not imparted by the single addition of each enzyme, was imparted. Also for pasta obtained by using durum wheat flour as a starting material, an effect of imparting "hardness" was exhibited in the same manner as for the cooked rice and udon noddle.

### Example 4

To 300 g of commercially available rice flour (manufactured by Mitake Shokuhin Kogyo Co., Ltd.), BE (3,650 U/g, manufactured by Nagase & Co., Ltd.) and/or AG (transglucosidase L "Amano", 608,000 U/g, manufactured by Amano Enzyme, Inc.) were/was added, followed by sufficient mixing. 240 g of water was added thereto, and kneading was carried out for 5 minutes using a kneader (manufactured by Kitchen Aid, Inc.). The dough obtained by kneading was steamed and kneaded by a microcomputer mochitsuki (pounding steamed rice into a dough for rice cakes) machine (Chikaramochi BE-SB10, manufactured by Zojirushi Corporation) under the automated mochitsuki function, into which 260 g of water for steaming was put in advance. The dough for rice cakes obtained by steaming and kneading was rolled to a thickness of about 1.5 mm using a pasta machine ("R.M.", manufactured by Imperia, Inc.), and then shaped into a disk having a diameter of 6 cm. The shaped dough was dried for 2 hours and 30 minutes under the conditions of 80°C and a humidity of 30% by a dryer "constant temperature and humidity chamber LH21-13P" (manufactured by Nagano Science Co., Ltd.), whereby a senbei cracker dough was prepared. The dried dough was baked at 210°C for 4 minutes and 30 seconds, whereby a senbei cracker was prepared. The amount of each enzyme was as shown in Table 10.

**Table 10**

| | Control group | Test group 38 | Test group 39 | Test group 40 | Test group 41 | Test group 42 |
|---|---|---|---|---|---|---|
| Addition amount of AG (U/g per g of flour) | - | 100 | - | - | 100 | 100 |
| Addition amount of BE (U/g per g of flour) | - | - | 3.7E-02 | 7.3E-02 | 3.7E-02 | 7.3E-02 |

The sensory evaluation of the senbei cracker was carried out after it was baked and then sufficiently cooled. The sensory evaluation was carried out with respect to the following three items: "hardness", "crunchability", and "crunchiness". In the sensory evaluation items, the "hardness" represents the strength of stress felt at the start of chewing the senbei cracker with the front teeth, the "crunchability" represents the strength of stress felt when the senbei cracker is chewed and crushed with the front teeth, and the "crunchiness" represents a sense of air bubbles felt when the senbei cracker is chewed and ground with the back teeth. In the same manner as in Example 1, the evaluation was carried out by 5 persons according to a scoring system with scores ranging from -2 to 2 in which the enzyme non-added group (control group) was given a score of 0. The detailed evaluation criteria are shown in Table 11. The results are shown in Table 12.

**Table 11**

| | -2 | -1 | 0 | 1 | 2 |
|---|---|---|---|---|---|
| Hardness | Very soft | Soft | There is no difference from control group | Hard | Very hard |
| Crunchability | Very difficult to crunch | Difficult to crunch | There is no difference from control group | Easy to crunch | Very easy to crunch |
| Crunchiness | Crunchiness is very low | Crunchiness is low | There is no difference from control group | Crunchiness is high | Crunchiness is very high |

**Table 12**

| | Control group | Test group 38 | Test group 39 | Test group 40 | Test group 41 | Test group 42 |
|---|---|---|---|---|---|---|
| Hardness | 0 | 0 | 0 | 0 | 1.5 | 2 |
| Crunchability | 0 | 0 | 1.25 | 1.5 | 1.5 | 2 |
| Crunchiness | 0 | 0.25 | 1.25 | 1.5 | 0 | 0 |

As shown in Table 12, in the case where BE was added singly, the "hardness" was comparable to that of the control group, and "crunchability" and "crunchiness" were imparted. On the other hand, in the case where AG was added singly, the "hardness" and "crunchability" were comparable to those of the control group, and a slight "crunchiness" was imparted. On the other hand, in the case where BE and AG were used in combination, "hardness", which was not imparted by the single addition of each enzyme, was imparted, and a higher "crunchability" than in the case where BE was added singly was imparted. By using BE and AG in combination, an effect of imparting "hardness" was exhibited, and moreover, an effect of imparting "crunchability" was synergistically improved. Also in the case of the senbei cracker which was a processed cooked rice product (dried food) obtained by using non-glutinous rice as a starting material, an effect of improving the physical properties was exhibited by using BE and AG in combination in the same manner as in the case of cooked rice.

### Example 5

700 g of commercially available glutinous rice (Kitoku Shinryo Co., Ltd.) was rinsed with tap water and soaked in tap water for 18 hours by adding tap water thereto in an amount twice the weight of the glutinous rice. When the glutinous rice was soaked in tap water, BE (3,650 U/g, manufactured by Nagase & Co., Ltd.) and/or AG (transglucosidase L "Amano", 608,000 U/g, manufactured by Amano Enzyme, Inc.) were/was dissolved and added thereto. After water was drained off, the soaked rice was steamed and kneaded by a microcomputer mochitsuki machine (Chikaramochi BE-SB10, manufactured by Zojirushi Corporation) under the automated mochitsuki function. 700 g of the dough for rice cakes obtained by steaming and kneading was packed in a pound cake mold (180 x 80 x h60 mm) and hardened by refrigeration for 27 hours. The hardened dough was cut to a thickness of 3 mm, and shaped into a size of about 3 cm x 2 cm. The shaped dough was dried for 17 hours and 30 minutes under the conditions of 38°C and a humidity of 55% by a dryer "constant temperature and humidity chamber LH21-13P" (manufactured by Nagano Science Co., Ltd.), whereby an okaki cracker dough was prepared. The dried dough was baked at 210°C for 4 minutes and 30 seconds, whereby an okaki cracker was prepared. The amount of each enzyme was as shown in Table 13.

**Table 13**

| | Control group | Test group 43 | Test group 44 | Test group 45 |
|---|---|---|---|---|
| Addition amount of AG (U/g per g of flour) | - | 50 | - | 50 |
| Addition amount of BE (U/g per g of flour) | - | - | 1.5E-01 | 1.5E-01 |

The sensory evaluation of the okaki cracker was carried out after it was baked and then sufficiently cooled. The sensory evaluation was carried out with respect to the following two items: "hardness" and "crunchiness". In the sensory evaluation items, the "hardness" represents the strength of stress felt at the start of chewing the okaki cracker with the front teeth, and the "crunchiness" represents a sense of air bubbles felt when the okaki cracker is chewed and ground with the back teeth. In the same manner as in Example 1, the evaluation was carried out by 5 persons according to a scoring system with scores ranging from -2 to 2 in which the enzyme non-added group (control group) was given a score of 0. The detailed evaluation criteria are shown in Table 14. The results are shown in Table 15.

**Table 14**

| | -2 | -1 | 0 | 1 | 2 |
|---|---|---|---|---|---|
| Hardness | Very soft | Soft | There is no difference from control group | Hard | Very hard |
| Crunchiness | Crunchiness is very low | Crunchiness is low | There is no difference from control group | Crunchiness is high | Crunchiness is very high |

**Table 15**

| | Control group | Test group 43 | Test group 44 | Test group 45 |
|---|---|---|---|---|
| Hardness | 0 | 0 | 0 | 2 |
| Crunchiness | 0 | 1.5 | 0.5 | 1.5 |

As shown in Table 15, in the case where BE was added singly, the "hardness" was not changed as compared with that of the control group, and a slight "crunchiness" was imparted. On the other hand, in the case where only AG was added, the "hardness" was not changed as compared with that of the control group, and "crunchiness" was imparted. In the case where BE and AG were used in combination, "hardness", which was not imparted by the single addition of each enzyme, was imparted, and by using BE and AG in combination, an effect of imparting "hardness" was exhibited. Also in the case of the okaki cracker which was a processed cooked rice product (dried food) obtained by using glutinous rice as a starting material, an effect of improving the physical properties was exhibited by using BE and AG in combination in the same manner as in the case of a senbei cracker obtained by using non-glutinous rice as a starting material.

### Example 6

240 g of commercially available strong wheat flour (manufactured by Nisshin Flour Milling, Inc.), 40 g of weak wheat flour (manufactured by Nisshin Flour Milling, Inc.), 15 g of sugar, 5 g of common salt, 7.5 g of butter, 3 g of dry yeast (manufactured by Nisshin Flour Milling, Inc.), 190 g of tap water, BE (3, 650 U/g, manufactured by Nagase & Co., Ltd.), and AG (transglucosidase L "Amano", 608, 000 U/g, manufactured by Amano Enzyme, Inc.) were put into a bread machine (Home Bakery PY-D532, Twinbird Corporation), which was set to the French bread making course, and processed to baking. The amount of each enzyme was as shown in Table 16.

**Table 16**

| Test group | Control group | Test group 46 | Test group 47 | Test group 48 |
|---|---|---|---|---|
| Addition amount of AG (U/g per g of flour) | - | - | 225 | 225 |
| Addition amount of BE (U/g per g of flour) | - | 4.4 | - | 4.4 |

The sensory evaluation of the bread was carried out after the bread was baked and then sufficiently cooled. The sensory evaluation was carried out with respect to the following three items: "hardness", "elasticity", and "meltability in mouth". In the sensory evaluation items, the "hardness" represents the strength of stress felt at the start of chewing the bread, the "elasticity" represents the strength of repulsive stress, that is, resilience when the bread is chewed, and the "meltability in mouth" represents the ease of swallowing such that the bread dissolves in the mouth without forming lumps. The evaluation was carried out by 5 persons according to a scoring system with scores ranging from -2 to 2 in which the enzyme non-added group (control group) was given a score of 0. The detailed evaluation criteria are shown in Table 17. The results are shown in Table 18.

**Table 17**

| | -2 | -1 | 0 | 1 | 2 |
|---|---|---|---|---|---|
| Hardness | Very soft | Soft | There is no difference from control group | Hard | Very hard |
| Elasticity | Elasticity is very low | Elasticity is low | There is no difference from control group | Elasticity is high | Elasticity is very high |
| Meltability in mouth | Meltability in mouth is very poor | Meltability in mouth is poor | There is no difference from control group | Meltability in mouth is good | Meltability in mouth is very good |

**Table 18**

| | Control group | Test group 46 | Test group 47 | Test group 48 |
|---|---|---|---|---|
| Hardness | 0 | -2 | -1.5 | 2 |
| Elasticity | 0 | 0 | 0 | 2 |
| Meltability in mouth | 0 | 1 | 1 | 1 |

As shown in Table 18, in the case where only BE or AG was added, the "hardness" was decreased, and softness and meltability in mouth were imparted. On the other hand, in the case where BE and AG were used in combination, the "hardness" and "elasticity" were significantly improved, and "hardness" and "elasticity" were imparted in the same manner as in the case of cooked rice.

### Industrial Applicability

According to the present invention, the quality of a starch-containing food can be improved, and therefore, the present invention is extremely useful in the field of foods.

## Claims

1. A method of producing a starch-containing food, **characterized by** adding a branching enzyme and an α-glucosidase to a starting material.

2. The method according to claim 1, wherein the addition amount of the branching enzyme is from 2.0×10⁻¹⁶ to 4.0×10⁵ U per g of the starting material, and the addition amount of the α-glucosidase is from 1.0×10⁻⁴ to 5.0×10⁷ U per g of the starting material.

3. The method according to claim 1 or 2, wherein the addition amount of the branching enzyme is from 4.0×10⁻²⁴ to 40 U per U of the α-glucosidase.

4. The method according to any one of claims 1 to 3, wherein the starch-containing food is a cooked rice food or a processed rice product, and the starting material is uncooked rice.

5. The method according to any one of claims 1 to 3, wherein the starch-containing food is a bread or a noodle.

6. An enzyme preparation for modifying a starch-containing food, comprising a branching enzyme and an α-glucosidase as active ingredients.

7. The enzyme preparation according to claim 6, wherein the content of the branching enzyme is from 4.0×10⁻²⁴ to 40 U per U of the α-glucosidase.
